# EUROPÄISCHE PATENTSCHRIFT

(11) **EP 0 852 138 B1**
(45) Veröffentlichungstag und Bekanntmachung des Hinweises auf die Patenterteilung: **17.04.2002**
(21) Anmeldenummer: 97121571.0
(22) Anmeldetag: 08.12.1997
(51) Int. Cl.: A61K 7/42

(54) **Kosmetische Zubereitungen**
Cosmetic compositions
Compositions cosmétiques

(30) Priorität: 16.12.1996 DE 19652300
(43) Veröffentlichungstag der Anmeldung: 08.07.1998
(73) Patentinhaber: Cognis Deutschland GmbH & Co. KG, 40589 Düsseldorf (DE)
(72) Erfinder: Ansmann, Achim, Dr., 40699 Erkrath (DE); Fabry, Bernd, Dr., 41352 Korschenbroich (DE)

(56) Entgegenhaltungen:
- WO-A-94/23693
- DE-A- 19 643 062

## Beschreibung

### Gebiet der Erfindung

Die Erfindung betrifft kosmetische Zubereitungen, vorzugsweise Selbstbräunungsmittel, enthaltend kationische Emulgatoren vom Esterquat-Typ und Dihydroxyaceton sowie die Verwendung von Esterquats zur Herstellung dieser Zubereitungen.

### Stand der Technik

In der modernen Leistungsgesellschaft wird von vielen die Bräunung der Haut mit Begriffen wie "jung" und "dynamisch" assoziiert. Für Verbraucher, die sich weder natürlicher noch künstlicher UV-Bestrahlung aussetzen können oder wollen, aber dennoch eine Hautbräunung wünschen, bietet die kosmetische Industrie Selbstbräunungspräparate an, die als Wirkstoff ganz überwiegend Dihydroxyaceton (DHA) enthält.

Ein Problem beim Einsatz von DHA besteht darin, daß die Stabilität in kosmetischen Produkten vielfach unzureichend ist und es zu unerwünschten Nebenreaktionen, wie z.B. zur Bildung von Formaldehyd oder Ameisensäure kommen kann; dies versucht man in der Regel durch Mikroverkapselung zu verhindern **[Euro Cosm. No.11, 26 (1995)]**. Aus der Europäischen Patentanmeldung **EP-A1 0689125** (L'Oréal) sind kosmetische O/W-Emulsionen mit DHA bekannt, die eine Teilchengröße im Bereich von 100 bis 1000 nm aufweisen. Gemäß der Europäischen Patentanmeldung **EP-A1 0715845** (L'Oréal) werden Selbstbräunungsmittel mit Dihydroxyaceton erhalten, indem man als Emulgator eine Mischung aus einem Alkylpolyglucosid und einem Fettalkohol einsetzt. Aus der Europäischen Patentanmeldung **EP-A1 0671159** (Shiseido) sind ferner Zusammensetzungen bekannt, die neben DHA, Ölkörper und Ethylenoxid/Propylenoxid-Blockpolymere enthalten.

Problematisch ist ferner die Tatsache, daß die Zubereitungen in aller Regel bei Raumtemperatur homogen sind und eine konstante Viskosität aufweisen, bei Erwärmung auf beispielsweise 35 bis 40°C jedoch schon nach kurzer Zeit eine irreversible Tendenz zur Entmischung zeigen und ihre Viskosität einbüßen. Wird ein solches Präparat angewendet, kommt es zu einer ungleichen Verteilung der selbstbräunenden Inhaltsstoffe auf der Haut, mit der Folge, daß der Bräunungseffekt unregelmäßig ausfällt. Es ist sofort klar, daß dies vom Verbraucher nicht toleriert werden kann.

Die Aufgabe der Erfindung hat somit darin bestanden, kosmetische Zubereitungen mit einem Gehalt an Dihydroxyaceton zur Verfügung zu stellen, die sich gleichzeitig durch eine hohe dermatologische Verträglichkeit, ein vorteilhaftes Hautgefühl sowie insbesondere eine hohe Lagerstabilität bei höheren Temperaturen auszeichnen. Ein wesentlicher Punkt in der Aufgabenstellung hat also darin bestanden, die Nachteile des Stands der Technik zu überwinden und sowohl die chemische Zersetzung des DHA als auch die ungleichmäßige Verteilung des Dihydroxyacetons bei Temperaturlagerung in den Zubereitungen zuverlässig zu verhindern.

### Beschreibung der Erfindung

Gegenstand der Erfindung sind kosmetische Zubereitungen, enthaltend
(a) Esterquats und
(b) Dihydroxyaceton.

Überraschenderweise wurde gefunden, daß unter Verwendung von Esterquats als kationischen Emulgatoren kosmetische Zubereitungen mit einem Gehalt an Dihydroxyaceton erhalten werden, die sich durch eine ausgezeichnete Lagerstabilität auch bei erhöhten Temperaturen auszeichnen. Auf diese Weise wird sichergestellt, daß die homogenen, viskosen Mittel auch bei direkter Sonneneinstrahlung ihre Stabilität nicht verlieren und vom Verbraucher leicht und zuverlässig angewendet werden können. Gleichzeitig wird eine hohe Stabilität des DHA beobachtet, ohne daß eine Mikroverkapselung erforderlich wäre.

### Esterquats

Unter der Bezeichnung "Esterquats" werden im allgemeinen quatemierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE-C1 4308794** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens. Surf.Det., 30, 186 (1993)**, M.Brock in **Tens.Surf.Det. 30, 394 (1993),** R.Lagerman et al. in **J.Am.Oil. Chem.Soc., 71, 97 (1994)** sowie I.Shapiro in **Cosm.Toil. 109, 77 (1994)** erschienen.

Die quatemierten Fettsäuretriethanolaminestersalze folgen der Formel **(I)**, 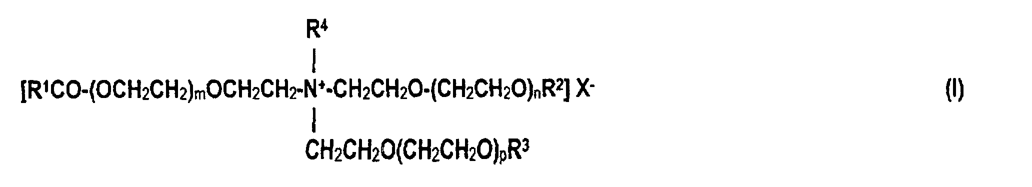 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quatemierten Ester können die Fettsäuren und das Triethanolamin im molaren Verhältnis von 1,1 : bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (lodzahl 0 bis 40) ab. Aus anwendungstechnischer Sicht haben sich quatemierte Fettsäuretriethanolaminestersalze der Formel (I) als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quatemierten Fettsäuretriethanolaminestersalzen kommen als Esterquats ferner auch quaternierte Estersalze von Fettsäuren mit Diethanolalkylaminen der Formel **(II)** in Betracht, 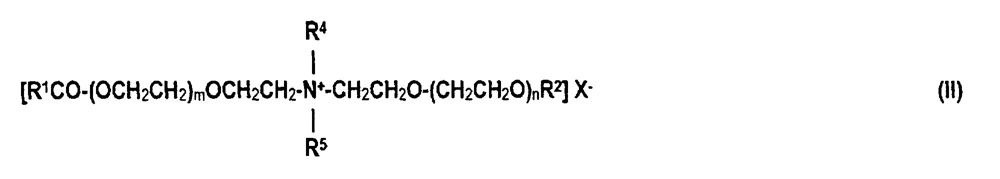 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quatemierten Estersalze von Fettsäuren mit 1,2-Dihydroxypropyldialkylaminen der Formel **(III)** zu nennen, 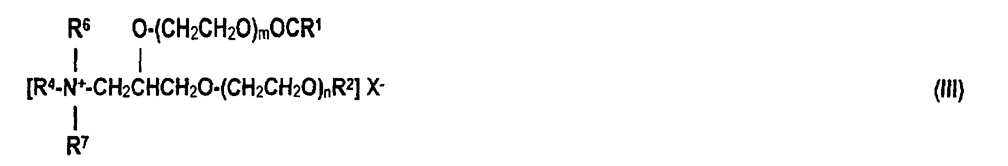 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III)**. Üblicherweise gelangen die Esterquats in Form 50 bis 90 Gew.-%iger alkoholischer Lösungen in den Handel, die bei Bedarf problemlos mit Wasser verdünnt werden können.

### Ölkörper

Die erfindungsgemäßen Zubereitungen enthalten als weitere Bestandteile vorzugsweise Ölkörper. Hierfür kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alko-holen (z.B. Finsolv® TN), Dialkylether, Siliconöle und/oder aliphatische bzw. naphthenische Kohlenwasserstoffe in Betracht. n einer bevorzugten Ausführungsform der Erfindung, enthalten die kosmetischen Zubereitungen
(a) 0,1 bis 10, vorzugsweise 1 bis 5 Gew.-% Esterquats,
(b) 0,01 bis 3, vorzugsweise 0,5 bis 2 Gew.-% Dihydroxyaceton und
(c) 1 bis 90, vorzugsweise 25 bis 75 Gew.-% Ölkörper,
mit der Maßgabe, daß sich die Mengenangaben gegebenenfalls mit Wasser und üblichen Hilfs- und Zusatzstoffen zu 100 Gew.-% ergänzen.

### Gewerbliche Anwendbarkeit

Mit Hilfe der kationischen Emulgatoren vom Esterquat-Typ werden zusammen mit Dihydroxyaceton auch bei höheren Temperaturen lagerstabile Zubereitungen, vorzugsweise Selbstbräunungsmittel in Form vom O/W-Emulsionen erhalten. Ein weiterer Gegenstand der Erfindung betrifft daher die Verwendung von Esterquats als Emulgatoren für Dihydroxyaceton zur Herstellung von Selbstbräunungsmitteln

Die erfindungsgemäßen Zubereitungen, wie beispielsweise Haarshampoos, Haarlotionen, Schaumbäder, Cremes, Lotionen oder Salben, können ferner als weitere Hilfs- und Zusatzstoffe milde Tenside, Co-Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, Insektenrepellentien, Farb- und Duftstoffe enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfo-succinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Co-Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-anlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinyl-imidazol-Polymere wie z.B. Luviquat® (BASF), Kondensationsprodukte von Polyglycolen und Aminen, quatemierte Kollagenpolypeptide wie beispielsweise Lauryldimonium hydroxypropyl hydrolyzed collagen (Lamequat®L/Grünau), quatemierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 22 52 840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quatemierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen.

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Zur Herstellung verschiedener O/W-Selbstbräunungsemulsionen wurden die Einsatzstoffe gemäß Tabelle 1 vermischt. Die erfindungsgemäßen Zubereitungen 1 bis 6 sowie die beiden Vergleichsemulsionen V1 und V2 wurden anschließend 2 Tage bei 35°C gelagert. Die Viskosität wurde nach der Brookfield-Methode in einem RVT-Viskosimeter (10 Upm, Spindel 1) bestimmt, die Beurteilung der Stabilität erfolgte visuell. Dabei bedeutet (+) eine stabile, homogene Emulsion und (-) Entmischung. Die Ergebnisse sind in Tabelle 1 zusammengefaßt.

**Tabelle 1**

| **Lagerstabilität von O/W-Selbstbräunungsemulsionen** | | | | | | | | |
|---|---|---|---|---|---|---|---|---|
| | **1** | **2** | **3** | **4** | **5** | **6** | **V1** | **V2** |
| Methylquatemierter Ditalgfettsäuretriethanolaminester, Methylsulfatsalz | 5,0 | 5,0 | 5,0 | 5,0 | 5,0 | 5,0 | - | - |
| Distearyldimethylammoniumchlorid | - | - | - | - | - | - | 5,0 | - |
| Cetearyl Glucoside (and) Cetearyl Alcohol | 5,0 | 5,0 | 5,0 | 5,0 | 5,0 | 5,0 | 5,0 | 5,0 |
| Ceteareth-10 | - | - | - | - | 5,0 | - | - | 5,0 |
| Ceteareth-20 | 5,0 | 5,0 | 5,0 | 5,0 | - | 5,0 | 5,0 | 5,0 |
| Dihydroxyaceton | 1,0 | 1,0 | 1,0 | 1,0 | 1,0 | 1,0 | 1,0 | 1,0 |
| Titandioxid | - | 1,0 | - | 0,5 | - | - | - | - |
| Tocopherol Acetate | - | - | 1,0 | 0,5 | - | - | - | - |
| Coco Glycerides | 10,0 | 10,0 | 10,0 | 10,0 | 10,0 | 10,0 | 10,0 | 10,0 |
| Oleyl Stearate | 5,0 | 5,0 | 5,0 | 5,0 | 5,0 | 5,0 | 5,0 | 5,0 |
| Mandelöl | 2,0 | 2,0 | 2,0 | 2,0 | 2,0 | 2,0 | 2,0 | 2,0 |
| Glycerin (86 Gew.-%ig) | 3,0 | 3,0 | 3,0 | 3,0 | 3,0 | 3,0 | 3,0 | 3,0 |
| Wasser | ad 100 | | | | | | | |

| ***Lagerstabilität*** | | | | | | | | |
|---|---|---|---|---|---|---|---|---|
| ***-Viskosität(sofort, 20°C) [mPas]*** | 1950 | 1950 | 1950 | 1950 | 1950 | 1950 | 1950 | 1950 |
| ***- Viskosität(nach 2 d, 35°C)[mPas]*** | 1900 | 1900 | 1900 | 1900 | 1850 | 1900 | 1400 | 1300 |
| **- *Stabilität (nach 2 d, 35°C)*** | + | + | + | + | + | + | - | - |

## Patentansprüche

1. Kosmetische Zubereitungen, enthaltend
(a) Esterquats und
(b) Dihydroxyaceton.

2. Zubereitungen nach Anspruch 1, **dadurch gekennzeichnet, daß** sie Esterquats der Formel (I) enthalten, 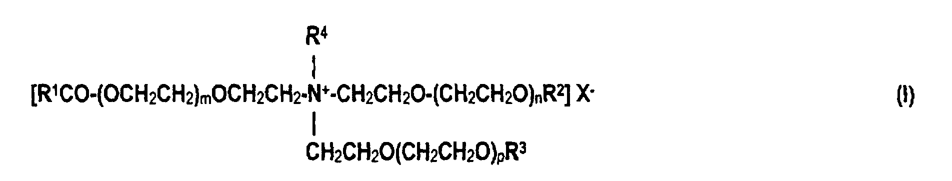 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Zubereitungen nach Anspruch 1, **dadurch gekennzeichnet, daß** sie Esterquats der Formel (II) enthalten, 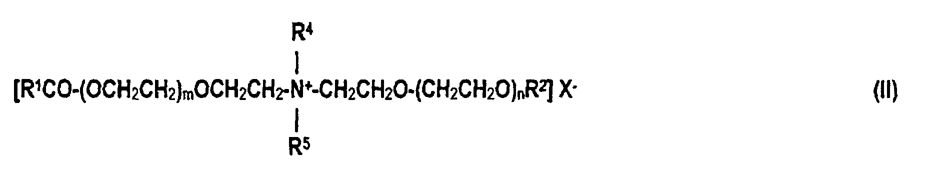 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Zubereitungen nach Anspruch 1, **dadurch gekennzeichnet, daß** sie Esterquats der Formel **(III)** enthalten, 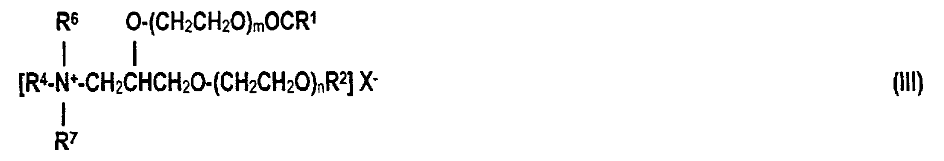 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Zubereitungen nach den Ansprüchen 1 bis 4, **dadurch gekennzeichnet, daß** sie Ölkörper enthalten, die ausgewählt sind aus der Gruppe, die gebildet wird von Guerbetalkoholen auf Basis von C₆-C₁₈-Fettalkoholen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estem von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, pflanzlichen Ölen, verzweigten primären Alkoholen, substituierten Cyclohexanen, linearen C₆-C₂₂-Fettalkoholcarbonaten, Guerbetcarbonaten, Estem der Benzoesäure mit linearen und/oder verzweigten C₆-C₂₂-Alkoholen, Dialkylethem, Siliconölen und/oder aliphatischen bzw. naphthenischen Kohlenwasserstoffen.

6. Kosmetische Zubereitungen, enthaltend
(a) 0,1 bis 10 Gew.-% Esterquats,
(b) 0,01 bis 3 Gew.-% Dihydroxyaceton und
(c) 1 bis 90 Gew.-% Ölkörper,
mit der Maßgabe, daß sich die Mengenangaben gegebenenfalls mit Wasser und üblichen Hilfsund Zusatzstoffen zu 100 Gew.-% ergänzen.

7. Verwendung von Esterquats als Emulgatoren für Dihydroxyaceton zur Herstellung von Selbstbräunungsmitteln.

## Claims

1. Cosmetic preparations containing
(a) esterquats and
(b) dihydroxyacetone.

2. Preparations as claimed in claim 1, **characterized in that** they contain esterquats corresponding to formula (I): 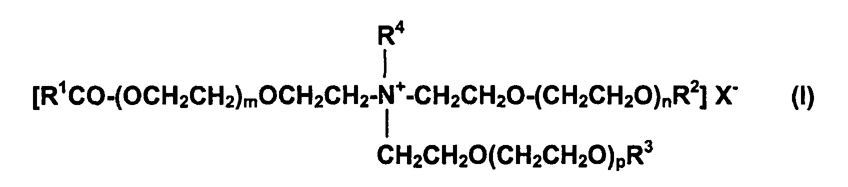 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate.

3. Preparations as claimed in claim 1, **characterized in that** they contain esterquats corresponding to formula **(II)**: 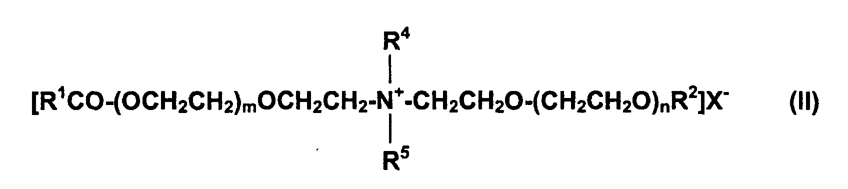 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate.

4. Preparations as claimed in claim 1, **characterized in that** they contain esterquats corresponding to formula **(III)**: 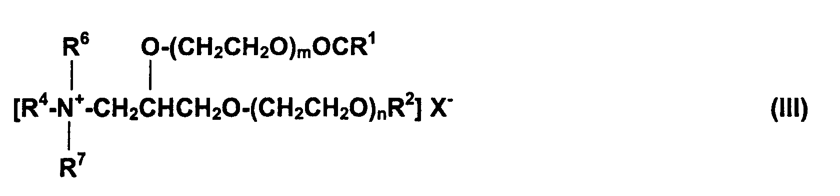 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate.

5. Preparations as claimed in claims 1 to 4, **characterized in that** they contain oil components selected from the group consisting of Guerbet alcohols based on C₆₋₁₈ fatty alcohols, esters of linear C₆₋₂₂ fatty acids with linear C₆₋₂₂ fatty alcohols, esters of branched C_{6ν13} carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, vegetable oils, branched primary alcohols, substituted cyclohexanes, linear C₆₋₂₂ fatty alcohol carbonates, Guerbet carbonates, esters of benzoic acid with linear and/or branched C₆₋₂₂ alcohols, dialkyl ethers, silicone oils and/or aliphatic or naphthenic hydrocarbons.

6. Cosmetic preparations containing
(a) 0.1 to 10% by weight esterquats,
(b) 0.01 to 3% by weight dihydroxyacetone and
(c) 1 to 90% by weight oil components, with proviso that the quantities shown add up to 100% by weight, optionally with water and typical auxiliaries and additives.

7. The use of esterquats as emulsifiers for dihydroxyacetone for the production of self-tanning preparations.

## Revendications

1. Formulations cosmétiques contenant
(a) des esterquate et
(b) de la dihydroryacétone.

2. Préparations selon la revendication 1,
**caractérisées en ce qu'**
elles contiennent des esterquats de formule (I), 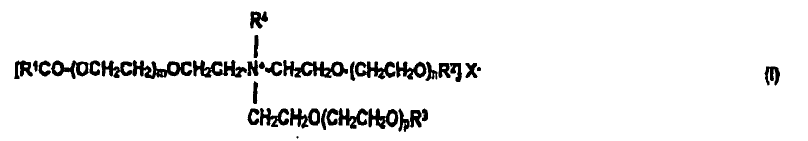 dans laquelle R¹CO représente un radical acyle comportant de 6 à 22 atomes de carbone, R² et R³ représentent indépendamment l'un de l'autre un hydrogène ou R¹CO, R⁴ représente un radical alkyle comportant de 1 à 4 atomes de carbone ou un groupe (CH₂CH₂O)_{q}H, m, n et p valent au total O ou des nombres allant de 1 à 12, q représente des nombres allant de 1 à 12 et X représente un halogénure, un sulfate d'alkyle ou un phosphate d'alkyle.

3. Préparations selon la revendication 1,
**caractérisées en ce qu'**
elles contiennent des esterquats de formule (II) 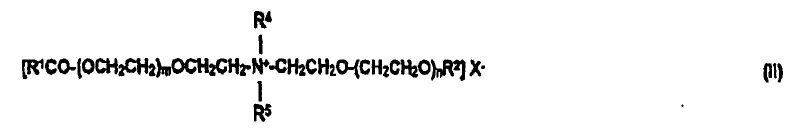 dans laquelle R¹CO représente un radical acyle comportant de 6 à 22 atomes de carbone, R² représente un hydrogène ou R¹CO, R⁴ et R⁵ représentent indépendamment l'un de l'autre des radicaux alkyle comportant de 1 à 4 atomes de carbone, m et n valent au total O et/ou des nombres allant de 1 à 12 et X représente un halogénure, un sulfate d'alkyle ou un phosphate d'alkyle.

4. Préparations selon la revendication 1,
**caractérisées en ce qu'**
elles contiennent des esterquats de formule (III) 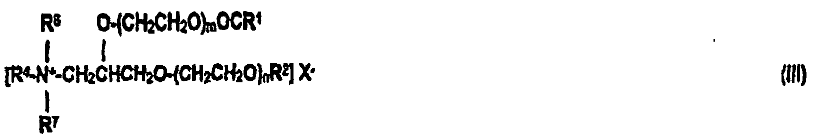 dans laquelle R¹CO représente un radical acyle comportant de 6 à 22 atomes de carbone, R² représente un hydrogène ou R¹CO, R⁴, R⁶ et R⁷ représentent indépendamment l'un de l'autre des radicaux alkyle comportant de 1 à 4 atomes de carbone, m et n valent au total O ou des nombres allant de 1 à 12 et X représente un halogénure, un sulfate d'alkyle ou un phosphate d'alkyle.

5. Préparation selon les revendications 1 à 4,
**caractérisées en ce qu'**
elles contiennent des corps huileux qui sont formés par les alcools de Guerbet à base d'alcools gras en C₆ à C₁₈, les esters d'acides gras linéaires en C₃ à C₆ linéaires avec des alcools gras en C₆ à C₂₂ linéaires, les esters d'acides carboxyliques en C₆ à C₁₃ ramifiés avec des alcools gras en C₆ à C₂₂ linéaires, les esters d'acides gras en C₆ à C₂₂ linéaires, avec des alcools ramifiés, les esters d'acides gras linéaires et/ou ramifiés avec des alcools polyvalents et/ou des alcools de Guerbet, les triglycérides à base d'acides gras en C₆ à C₁₈, les mélanges de mono-, di/triglycérides liquides à base d'acides gras en C₆ à C₁₈, les esters d'alcools gras en C₆ à C₂₂ et/ou les alcools de Guerbet avec des acides carboxyliques aromatiques, les huiles végétales, les alcools primaires ramifiés, les cyclohexanes substitués, les carbonates d'alcools gras en C₆ à C₂₂ linéaires, les carbonates de Guerbet, les esters d'acide benzoïque avec des alcools en C₆ à C₂₂ linéaires ou ramifiés, les éthers de dialkyle, les huiles de silicone et/ou les hydrocarbures aliphatiques ou selon les cas naphténiques.

6. Préparations cosmétiques contenant
(a) de 0,1 à 10 % en poids d'esterquats.
(b) de 0,01 à 3 % en poids de dihydroxyacétone et
(c) de 1 à 90 % en poids de corps huileux,
sous réserve que les indications quantitatives sont les cas échéant complétées avec de l'eau et des adjuvants et additifs habituels à 100 % en poids.

7. Utilisation d'esterquats comme émulsifiants pour la dihydrogyacétone en vue de la préparation d'agents d'autobronzage.
